Europäisches Patentamt

European Patent Office

Office européen des brevets

(19)

(11) Veröffentlichungsnummer: **0 247 103**
**B1**

(12) **EUROPÄISCHE PATENTSCHRIFT**

(45) Veröffentlichungstag der Patentschrift: **02.01.91**

(51) Int. Cl.⁵: **A 61 B 3/10**

(21) Anmeldenummer: **86906781.9**

(22) Anmeldetag: **24.11.86**

(86) Internationale Anmeldenummer:
**PCT/DE86/00478**

(87) Internationale Veröffentlichungsnummer:
**WO 87/03188 04.06.87 Gazette 87/12**

(54) **ANORDNUNG ZUR MESSUNG DER AUGEN-LINSEN-TRÜBUNG.**

(30) Priorität: **29.11.85 DE 3542167**

(43) Veröffentlichungstag der Anmeldung:
**02.12.87 Patentblatt 87/49**

(45) Bekanntmachung des Hinweises auf die
Patenterteilung:
**02.01.91 Patentblatt 91/01**

(84) Benannte Vertragsstaaten:
**AT CH FR GB IT LI NL SE**

(56) Entgegenhaltungen:
**DE-A-3 117 699**
**US-A-4 327 973**

(73) Patentinhaber: **LOHMANN, Wolfgang**
**Petersweiher 20**
**D-6300 Giessen (DE)**

(72) Erfinder: **LOHMANN, Wolfgang**
**Petersweiher 20**
**D-6300 Giessen (DE)**

Courier Press, Leamington Spa, England.

EP 0 247 103 B1

## Beschreibung

Die Erfindung betrifft eine Anordnung zur in-vivo-Messung der Augen-Linsen-Trübung, insbesondere der cataracta nuclearis, entsprechend dem Oberbegriffe des Anspruchs 1.

Eine solche Anordnung ist aus einer Druckschrift der Fa. COHERENT, Medical Group, USA Lit. No. 3012 IOM, bekannt. Ein automatisches Fluoreszenzspektrometer mit Auswerteeinrichtung zur Analyse des Fluoreszenzlichtes wird in der DE-OS 29 22 788 beschrieben.

Die cataracta nuclearis ist eine häufig anzutreffende Augenlinsentrübung bei älteren Menschen. Bei ihr nimmt die Dichte und Trübung des zentralen Teils der Linse mit fortschreitender Erkrankung zu. Parallel dazu erfolgt eine Verfärbung der Linse von hellgelb bis dunkelbraun. Diese Veränderungen in der Linse führen zu einem teilweisen Verlust des Sehvermögens oder sogar zur Erblindung. Trotz intensiver Untersuchungen ist bis jetzt nicht viel über die Ursache oder die molekularen Mechanismen der Katarakt-Bildung bekannt. In Appl.Opt. 10, S.459 ff. (1971), wird beschrieben, daß die Bildung von protein-Aggregaten mit hohem Molekulargewicht für die Linsentrübung verantwortlich sein soll. Die Färbung wird dagegen dem Vorhandensein photochemisch induzierter Chromophoren zugeschrieben (S. Lerman in "Altern der Linse", S,139 ff, Symposium über die Augenlinse, Straßburg (1982)).

Die Linsentrübung wird gewöhnlich mittels einer konventionellen Spaltlampen-Untersuchung festgestellt. Hierbei können Aussagen über schwerpunktmäßige Lokalisationen von Trübungen sowie über den Reifegrad der Trübung gemacht werden. Beide Aussagen sind in erheblichem Umfang von der subjektiven Einschätzung des Befundes abhängig. Für eine objektive Festlegung einer Linsentrübung steht bisher kein in der Praxis im Routinebetrieb einsatzfähiges Gerät zur Verfügung.

Untersuchungen der Fluoreszenz-Intensität einzelner Chromophore sind mittels einer modifizierten Scheimpflugkamera durchgeführt worden. Bei dieser Methode wird mit einem relativ breiten Wellenlängenbereich im UV-Gebiet (300—400nm) die Fluoreszenz angeregt und bei zwei diskreten Wellenlängen (440nm und 520nm) die Fluoreszenz-Intensität gemessen. Detaillierte Aussagen über den Grad der cataracta nuclearis können leider auch mit dieser Methode nicht gemacht werden.

Zur Verbesserung der Nachweisempfindlichkeit von Methoden für den Nachweis minimaler, jedoch bedeutender Veränderungen in biologischen Systemen sind in den letzten Jahren die verschiedensten Label oder Tracer eingeführt worden. Neben den radioaktiven Labels sind dies im wesentlichen die Fluoreszenz-Labels. Alle diese Labels sind nicht körpereigen und müssen entweder injiziert oder oral verabreicht werden. Auch wenn sie nur in Spuren (Tracer)-Mengen gegeben werden, so beeinflussen sie doch das relevante biologische System nachhaltig.

Für Katarakt-Untersuchungen wurde auf dieser Basis in den letzten Jahren die Fluorophotometrie mittels Fluorescein als Label entwickelt (Firma COHERENT, siehe auch DE-A-3,117,699). Neben dem Eingriff in das biologische System hat diese Methode den Nachteil, daß die Anregungswellenlänge von der Fluoreszenz-Wellenlänge des Fluoresceins abhängig ist und der Patient sich nach der Untersuchung für längere Zeit nicht dem Sonnenlicht aussetzen darf, da seine Augen durch das Fluorescein sehr lichtempfindlich geworden und mögliche Schäden nicht auszuschließen sind.

Fluorescein wird ebenfalls zur Untersuchung von Blut-Retina und Blut-Wasser Barrieren oder zur Darstellung der Mikrokapillaren des Augenhintergrundes benutzt. Trotz der oben geäußerten Bedenken ist es die gängige Methode, da zur Zeit keine besseren Verfahren zur Verfügung stehen.

Da die Linsentrübung allmählich erfolgt, bemerken in den meisten Fällen die patienten anfänglich nichts von der Linsentrübung. Erst in relativ fortgeschrittenem Stadium wird der Arzt aufgesucht. Die genannten Untersuchungsmethoden können auf keinen Fall eine exakte Abstufung der Linsentrübung geben. Üblicherweise werden heute vier Stadien für ihre Klassifikation benutzt, deren Zuordnung vom behandelnden Arzt nicht immer eindeutig erfolgt. Ein wesentlicher Grund dafür liegt darin, daß für die einzelnen Stadien keine quantitativen Werte vorgegeben werden können und somit die Einteilung subjektiv und willkürlich geschieht. Zu einer genauen Bestimmung des Krankheitsverlaufs ist es deshalb unbedingt erforderlich, quantitative Aussagen machen zu können, d.h. einen direkten Bezug der Erkennungskriterien zu den Veränderungen in der Katarakt-Linse herzustellen. Besonderer Wert muß dabei auf eine Früherkennung gelegt werden, um eine weitere Ausbildung der Krankheit zu verhindern oder wenigstens zu verzögern. Die konventionelle Spaltlampen-Untersuchung ist hierfür viel zu unempfindlich.

Der Erfindung lag daher die Aufgabe zugrunde, eine Anordnung anzugeben, mit der es möglich ist, auch geringfügige Linsenveränderungen so früh wie möglich zu erkennen und darüber hinaus den Grad der Katarakt-Bildung innerhalb einer die Linsenveränderungen beschreibenden Skala exakt bestimmen zu können. Die Anordnung sollte weitgehend aus bekannten und bei der Augenuntersuchung bewährten Bauteilen aufgebaut sein.

Diese Aufgabe wird bei einer Anordnung der eingangs genannten Art erfindungsgemäß durch die im kennzeichnenden Teil des Anspruchs 1 genannten Maßnahmen gelöst. Vorteilhafte Ausgestaltungen ergeben sich aus den Merkmalen der Ansprüche 2 bis 9.

Der Erfindungsgegenstand kann mit folgenden unabhängig voneinander gemachten Beobachtungen in Zusammenhang gebracht werden, wobei zusätzlich eine Aussage über die Ursachen der Katarakt-Bildung gewonnen werden kann.

Bei früheren Untersuchungen konnte festgestellt werden, daß bei geringen Anomalien im menschlichen Gewebe-System im Elektronenspin-Resonanz-Spektrum der relevanten Gewebe ein zusätzliches Signal auftritt, das dem Ascorbyl-Radikal zugeordnet werden konnte. Da in intakten biologischen Systemen Ascorbinsäure (Vitamin C) fast nur im reduzierten Zustand vorliegt, handelt es sich also bei den untersuchten Anomalien um eine Stoffwechselstörung, die das Vitamin C Redox-Gleichgewicht beeinflußt und zu einer Oxidation des Vitamin C von der Ascorbinsäure über das Ascorbyl-Radikal zur Dehydroascorbinsäure führt. Bei fortschreitender Erkrankung überwiegt der Oxidationsprozeß, als Folge dessen auch die Dehydroascorbinsäure oxidiert wird. Das führt zu oxidativen Zerfallsprodukten des Vitamin C, z.B. Diketogulonsäure bis hin zum Methylglyoxal.

Bei der Untersuchung der Vitamin C-Oxidations-Mechanismen wurde die interessante Feststellung gemacht, daß eine Vitamin C-Lösung, die nach frischem Ansetzen transparent ist, sich im Laufe der Zeit (Tage bis Wochen) über gelb bis dunkelbraun verfärbt und mit zunehmender Verfärbung ein charakteristisches Fluoreszenz Spektrum ergibt. Katarakt-Linsen durchlaufen eine ähnliche Verfärbung. Da es bekannt ist, daß die Augenlinse eine hohe Konzentration an Ascorbinsäure hat, wurde auf Grund des ähnlichen Verfärbungsverhaltens auch das Fluoreszenzverhalten der Linsen untersucht. In überraschender Weise wurde dabei gefunden, daß eine exakte Parallelität im Fluoreszenz-Verhalten der Katarakt Linsen und der Vitamin C-Lösungen vorliegt. Bei monochromatischer Anregung zwischen 350—500nm und Registrierung der Fluoreszenz über einen bestimmten Spektralbereich bis etwa 650nm zeigen frisch angesetzte Vitamin C-Lösungen keine Fluoreszenz; dies trifft auch für gesunde Linsen zu, die zwar bei 350nm Anregung bereits eine geringfügige Eigenfluoreszenz aufweisen, die aber bei längerwelliger Anregung unterbleibt und im übrigen keine charakteristischen Eigenheiten erkennen läßt. Die spezifische Fluoreszenz bildet sich erst mit zunehmender Verfärbung weiter aus und ist sowohl hinsichtlich Intensität als auch Lage des Fluoreszenzmaximums (Wellenlänge) charakteristisch für den jeweiligen Verfärbungsgrad. Da die Fluoreszenz-Messung eine der empfindlichsten Meßmethoden darstellt, können durch die erfindungsgemäß Anordnung bereits in einem sehr frühen Stadium Veränderungen in der Linse bei Katarakt-Bildung nachgewiesen und aufgrund des Verteilungsmusters der Fluoreszenz-Intensitäten minutiöse Unterschiede zwischen den einzelnen Katarakt-Stadien nachgewiesen werden.

Die bei der Untersuchung von Vitamin C-Lösungen gefundenen Ergebnisse lassen den Schluß zu, daß auch der zunehmenden Ausbildung der Linsentrübung und -verfärbung eine zunehmende Oxidation der Ascorbinsäure zugrunde liegt. Diese Erkenntnis begründet die Vorteile des erfindungsgemäßen Verfahrens. Dem Patienten brauchen keine nicht-körpereigenen fluoreszierenden Substanzen verabfolgt zu werden. Es wird die Primär-Fluoreszenz einer körpereigenen Substanz gemessen, deren Oxidationsgrad dem Entwicklungsstand des Katarakts entspricht. Die Verfügbarkeit mehrerer Fluoreszenz-Banden gibt durch Intensitätsvergleiche darüber hinaus weitere Detail-Informationen, die sicherlich in der Zukunft für therapeutische Zwecke dienlich sind, da das Fluoreszenz-Spektrum ebenfalls den Fortschritt - und Erfolg - in der Behandlung aufzeigt. Das Verfahren ist sehr spezifisch. In dem untersuchten Wellenlängenbereich (350—500nm für die Anregung; 380—650nm für die Registrierung) treten kaum Störfluoreszenzen auf. Es kann bereits eine Konzentration von etwa 1µM oxidiertes Vitamin C nachgewiesen werden.

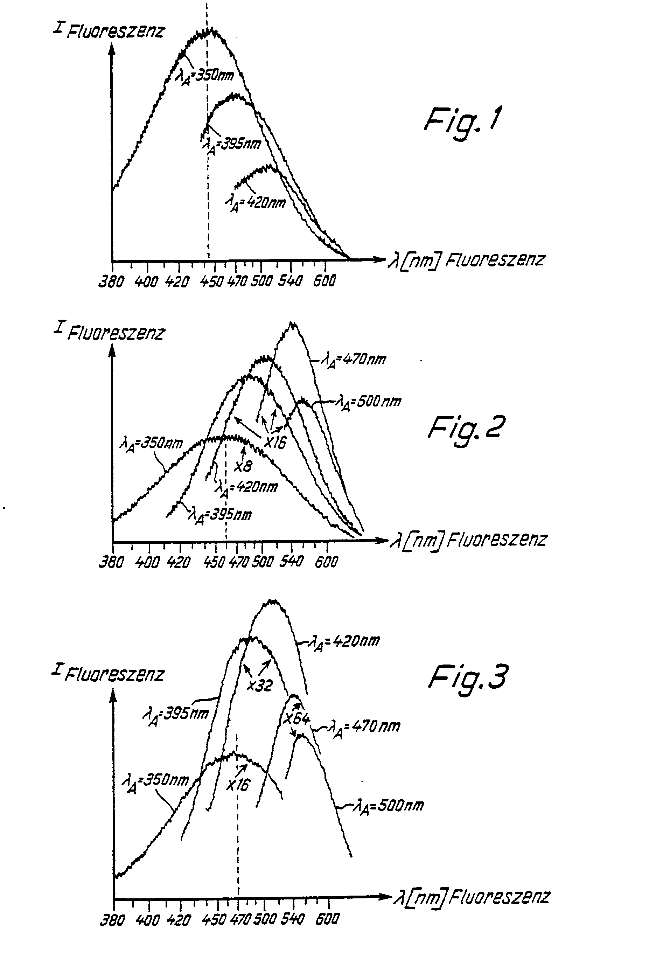

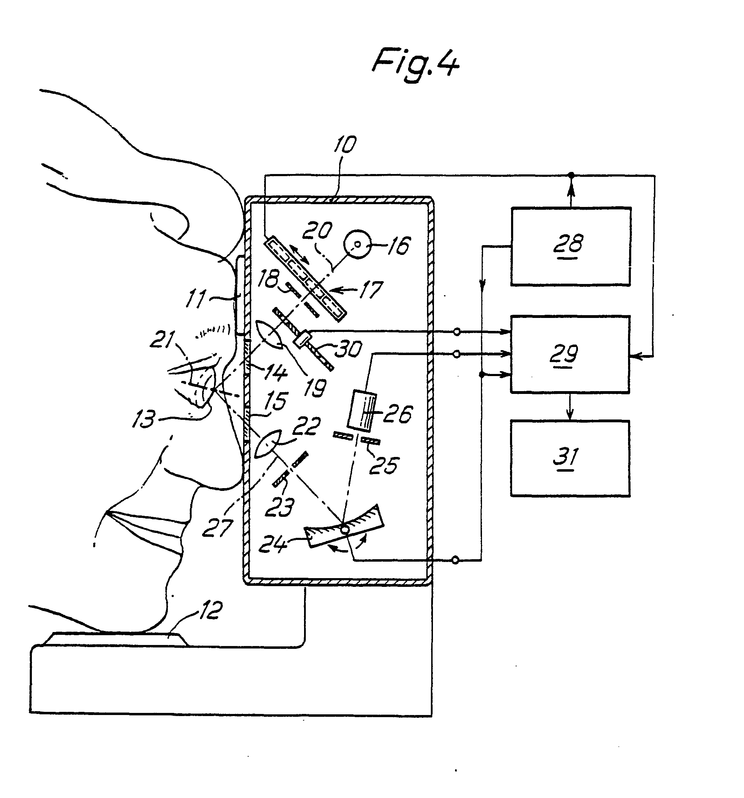

Die Fluoreszenzspektren für verschiedene Anregungswellenlängen bei unterschiedlich fortgeschrittenen Katarakt-Stadien sind in den Figuren 1 bis 3 dargestellt. Fig. 4 zeigt eine schematische Darstellung einer geeigneten Meßvorrichtung.

Die in Fig. 1 dargestellten Meßkurven wurden bei der Messung an einer gelblich verfärbten Linse gewonnen. Es entstehen deutlich ausgeprägte Fluoreszenzspektren für die Anregungswellenlängen $\lambda_A$=350nm, $\lambda_A$=395nm und $\lambda_A$=420nm. Mit steigender Anregungswellenlänge $\lambda_A$ nimmt die Intensität der Fluoreszenzspektren ab. Für größere Anregungswellenlängen lassen sich in diesem Stadium der Katarakt-Bildung noch keine Fluoreszenzspektren nachweisen. Andererseits kann bereits aus dem ersten Auftreten eines Fluoreszenzspektrums für $\lambda_A$=350nm eine beginnende Katarakt-Bildung erkannt werden. Die dem Maximum des Fluoreszenzspektrums für $\lambda_A$=350nm zugeordnete Wellenlänge liegt in diesem Stadium bei 445nm. Für geringere Grade der Katarakt-Bildung verschiebt sich das Maximum zu längeren Wellenlängen, wie aus den nachfolgenden Darstellungen ableitbar ist.

Der in Fig. 2 dargestellten Messung liegt eine bereits bräunlich verfärbte Augenlinse zugrunde. Die Intensität der Fluoreszenz nimmt insgesamt stark zu. Für das am weitesten links liegende, zu $\lambda_A$=350nm gehörende Fluoreszenzspektrum wurde der Auftragungsmaßstab im Vergleich zu Fig. 1 um den Faktor 8 gedämpft. Die Intensitäten der weiteren Fluoreszenzspektren wurden um den Faktor 16 gedämpft, um eine zu Fig. 1 vergleichbare Darstellung zu erhalten. Auffällig ist die Anregung der längerwelligen Fluoreszenzspektren zu $\lambda_A$=470nm und $\lambda_A$=500nm, deren Intensitäten die des kurzwelliger angeregten Fluoreszenzspektrums bei weitem übersteigen. Das Maximum des Fluoreszenzspektrums zu $\lambda_A$=350nm liegt jetzt bei etwa 460nm.

Die in Fig. 2 bereits erkennbare Tendenz setzt sich in Fig. 3 fort. Der in dieser Figur dargestellten Messung liegt eine bereits dunkelbraun verfärbte Katarakt-Linse zugrunde. Für den Vergleich der dargestellten Fluoreszenzspektren muß beachtet werden, daß die Intensität des mit $\lambda_A$=350nm angeregten Fluoreszenzspektrums im Vergleich zu der Darstellung in Fig. 1 um den Faktor 16 reduziert wurde, während die beiden nachfolgenden Fluoreszenzspektren zu $\lambda_A$=395nm und

$\lambda_A$=420nm um den Faktor 32, die beiden rechts auftretenden Fluoreszenzspektren zu $\lambda_A$=470nm und $\lambda_A$500nm sogar um den Faktor 64 reduziert wurden, um eine vergleichbare Darstellung innerhalb derselben Figur zu finden. Das Maximum des Fluoreszenzspektrums zu $\lambda_A$=350nm hat sich weiterhin in den längerwelligen Bereich zu $\lambda$=470nm verschoben.

Die in den Fig. 1 bis 3 dargestellten Messungen machen folgendes deutlich. Eine beginnende Katarakt-Bildung läßt sich sicher an der Fluoreszenz bei $\lambda_A$=350nm erkennen. Durch Reihenmessungen bei unterschiedlichen Verfärbungsgraden der cataracta nuclearis läßt sich eine Wellenlängenskala aufstellen, die den visuell wahrgenommenen Farbeindrücken zugeordnet ist.

Mit fortschreitender Katarakt-Bildung, d.h. zunehmender Verfärbung, setzt die Fluoreszenz bei längerwelliger Anregung nach und nach ein und steigt dann wesentlich stärker an als die bei kurzwelliger Anregung. Jedes Stadium der Katarakt-Bildung ist daher neben der Verschiebung des Fluoreszenzmaximums bei kurzwelliger Anregung auch durch das Intensitätsverhältnis der Fluoreszenzmaxima zu verschiedenen Anregungswellenlängen gekennzeichnet. Die Wellenlängenskala kann daher in vorteilhafter Weise, insbesondere im Bereich bereits visuell erkennbarer Katarakt-Bildung, durch ein oder mehrere typische Intensitätsverhältnisse ergänzt werden. Da die Intensitätsveränderungen entsprechend den Darstellungen in Fig. 1—3 wesentlich deutlicher sind als die Wellenlängenverschiebungen der Maxima der Fluoreszenzspektren, gestattet eine nach diesen Kriterien aufgestellte Skala eine noch feinere Unterteilung für die quantitative Angabe des Grades der Katarakt-Bildung.

Die in den Figuren 1 bis 3 dargestellte analoge Aufzeichnung der Fluoreszenzspektren läßt sich mit Hilfe bekannter elektronischer Schaltungen digitalisieren und kann dann einem Rechner zur Auswertung zugeführt werden.

In Fig. 4 ist schematisch eine Anordnung dargestellt, die aus an sich bekannten Bauteilen zusammengestellt ist, jedoch in besonders vorteilhafter Weise die Durchführung des erfindungsgemäßen Verfahrens ermöglicht. Der optische Teil der Meßeinrichtung ist in einem Gehäuse 10 untergebracht. Dieses weist verstellbare Anlage- und Auflageflächen 11, 12 auf, an denen der Kopf des Patienten so gestützt werden kann, daß sich die zu untersuchende Augen-Linse 13 am vorgesehenen Meßort befindet.

Das Gehäuse weist vorzugsweise zwei Öffnungen 14, 15 auf, durch die einmal ein Spaltbild auf die Augenlinse 13 projiziert und zum anderen das angeregte Fluoreszenlicht aufgenommen wird. Durch eine weitere, nicht dargestellte Öffnung oder mit Hilfe geeigneter Strahlenteilung im Beleuchtungsstrahlengang könnte ergänzend noch die Lage des Spaltbildes auf der Augen-Linse beobachtet werden.

Der Beleuchtungsstrahlengang enthält eine Lichtquelle 16, deren Emissionsspektrum die geforderten Anregungswellenlängen in ausreichender Intensität enthält, wie z.B. eine Xenon-Hochdruck-Lampe. Mit Hilfe eines nachgeschalteten Monochromators (nicht dargestellt) oder einer Reihe, auf einem Schieber 17 angeordneter, Interferenzfilter wird eine monochromatische Beleuchtung des Spaltes 18 erzeugt. Der Spalt wird über die Optik 19 durch die Öffnung 14 hindurch auf die zu untersuchende Linse 13 abgebildet. Die optische Achse 20 des Anregungsstrahlenganges steht vorzugsweise etwa unter 60° zur Achse 21 der Augenlinse, um Störfluoreszenzen an nicht interessierenden Gewebeteilen möglichst zu unterdrücken.

Das an der Augenlinse angeregte Fluoreszenlicht wird über eine Optik 22 durch die Öffnung 15 hindurch aufgenommen und einem registrierenden Spektralphotometer zugeleitet. Dieses besteht z.B. aus einem Eintrittsspalt 23 und einem steuerbar verstellbaren Beugungsgitter 24, das den Eintrittsspalt 23 auf einen Austrittsspalt 25 abbildet. Dem Austrittsspalt 25 nachgeordnet ist ein fotoelektrischer Empfänger 26. Die optische Achse 27 des Nachweisstrahlenganges steht vorzugsweise unter 90° zur optischen Achse 20 des Anregungsstrahlenganges. Die durch den Anregungs- und Nachweisstrahlengang aufgespannte Ebene kann unter beliebigem Winkel zur Augenlinse angeordnet werden.

Die Verstellung des Filterschiebers 17 erfolgt vorzugsweise mit Hilfe einer Programmsteuerung 28 über einen nicht dargestellten Motor. Sie kann aber auch manuell geschehen. Auch das Beugungsgitter 24 wird zur Aufnahme des Fluoreszenzspektrums vorzugsweise motorisch verstellt, wobei der aufzunehmende Spektralbereich ebenfalls über die Programmsteuerung 28 vorgegeben wird.

Das von dem fotoelektrischen Empfänger 26 abgegebene Signal wird einer Signalverarbeitungsschaltung 29 mit Rechner zur Auswertung zugeleitet. Zur besseren Unterdrückung des Anregungslichtes im Nachweisstrahlengang und zur Verbesserung des Signal-/Rauschverhältnisses im Meßsignal ist in dem Anregungsstrahlengang ein Chopper 30 eingefügt. Die Signalauswertung wird mit der Unterbrecherfrequenz des Choppers 30 so gesteuert, daß das Fluoreszenzsignal nur während der Zeit aufgenommen wird, in der der Anregungsstrahlengang unterbrochen ist.

Durch die zusätzliche Eingabe der programmsteuersignale für die Anregungs-Filterstellung und die Fluoreszenzspektrum-Registrierung in die Signalverarbeitungsschaltung 29 ist ein automatischer Meßablauf zur objektiven Bestimmung des Grades der Katarakt-Bildung in der Augen-Linse 13 möglich. Der der Schaltung 29 zugeordnete Rechner bestimmt Lage und Intensität der Maxima der aufgenommenen Fluoreszenzspektren, ermittelt die Intensitätsverhältnisse der den Maxima der Fluoreszenzspektren zugeordneten Wellenlängen $\lambda_{Max}$, vergleicht diese mit der die Katarakt-Bildung beschreibenden Skala und bringt den ermittelten Wert auf einem Anzeigegerät 31 zur Darstellung.

## Patentansprüche

1. Anordnung zur in-vivo-Messung der Augen-Linsen-Trübung, insbesondere der cataracta nuclearis, mit einer Vorrichtung zur ortsfesten Lagerung des zu untersuchenden Auges, einer Projektionsvorrichtung zur Projektion eines Spaltbildes auf die Augenlinse, einer Meß-Vorrichtung zur Messung des an der Linse angeregten Fluoreszenzlichtes, einer Auswerteeinrichtung zur Analyse des Fluoreszenzlichtes und einer Anzeigeeinrichtung für die Meßwerte, dadurch gekennzeichnet, daß

a) die Projektionsvorrichtung (16—19) Mittel zur Erzeugung eines monochromatischen Anregungsstrahlenbündels enthält, dessen Wellenlänge $\lambda_A$ zwischen 350nm und 500nm liegt,

b) die Meßvorrichtung ein registrierendes Spektralphotometer (22—26) enthält, das zu jeder Anregungswellenlänge $\lambda_A$ im dazu längerwelligen Bereich zwischen minimal 380nm und maximal 650nm ein Fluoreszenzspektrum aufnimmt,

c) die Auswerteeinrichtung (29) die der Maximalintensität 1 des registrierten Fluoreszenzspektrums zugeordnete Wellenlänge $\lambda_{max}$ ermittelt,

d) die Auswerteeinrichtung (29) einen Speicher mit einer Werteskala für die Augen-Linsen-Trübung enthält, der eine empirisch ermittelte Wertetabelle der Meßparameter $\lambda_A$ und $\lambda_{max}$ zugeordnet ist, und

e) die Auswerteeinrichtung (29) den gesuchten Wert der Augen-Linsen-Trübung durch Vergleich der aktuellen Meßparameter $\lambda_A$, $\lambda_{max}$ mit der Wertetabelle des Speichers ermittelt.

2. Anordnung nach Anspruch 1, dadurch gekennzeichnet, daß

a) eine Programmsteuerung (28) vorgesehen ist, die nacheinander zwei unterschiedliche Anregungswellenlängen $\lambda_{A1}$, $\lambda_{A2}$ einstellt und die Analyse der zugehörigen Fluoreszenzspektren durch die Auswerteeinrichtung (29) veranlaßt,

b) die Auswerteeinrichtung (29) die den Maximalintensitäten $I_1$, $I_2$ zugeordneten Wellenlängen $\lambda_{max1}$ und $\lambda_{max2}$ ermittelt und zusätzlich aus den Maximalintensitäten $I_1$, $I_2$ das Intensitätsverhältnis $I_1/I_2$ bildet, und

c) die in dem Speicher abgelegte Wertetabelle zusätzlich empirisch ermittelte Intensitätsverhältnisse für entsprechende Anregungswellenlängen enthält.

3. Anordnung nach Anspruch 2, dadurch gekennzeichnet, daß eine dritte Anregungswellenlänge $\lambda_{A3}$ vorgesehen ist, deren Fluoreszenzspektrum eine Maximalintensität $1_3$ aufweist, daß die Programmsteuerung nacheinander die drei Anregungswellenlängen $\lambda_{A1}$, $\lambda_{A2}$, $\lambda_{A3}$ einstellt, daß die Auswerteeinrichtung aus den zugehörigen Maximalintensitäten $I_1$, $I_2$, $I_3$ Intensitätsverhältnisse $I_1/I_2$ und $I_2/I_3$ bildet, und daß die Wertetabelle des Speichers dementsprechend ergänzt ist.

4. Anordnung nach einem der vorhergehenden Ansprüche, dadurch gekennzeichnet, daß als Anregungswellenlänge $\lambda_A$ mindestens eine der Wellenlängen 350nm, 395nm, 420nm, 470nm oder 500nm dient und die zugehörigen Fluoreszenzspektren in den Wellenlängenbereichen 380—650nm, 430—650nm, 460—650nm, 490—650nm und 520—650nm liegen.

5. Anordnung nach einem der vorhergehenden Ansprüche, dadurch gekennzeichnet, daß dem Spektralphotometer eine Schaltungsanordnung zur Digitalisierung der gemessenen Fluoreszenzspektren zugeordnet ist.

6. Anordnung nach einem der vorhergehenden Ansprüche, dadurch gekennzeichnet, daß eine Regelschaltung die Intensität des Anregungsstrahlenbündels in Abhängigkeit von der Intensität des Fluoreszenzlichtes einstellt.

7. Anordnung nach einem der vorhergehenden Ansprüche, dadurch gekennzeichnet, daß die optische Achse (20) des Projektionsstrahlenganges gegenüber der Achse (21) des Auges und der Achse des Nachweisstrahlenganges (27) so neigbar ist, daß an vor und hinter der Augenlinse (13) liegenden Gewebeschichten angeregte Störfluoreszenzen möglichst weitgehend reduziert werden.

8. Anordnung nach einem der vorhergehenden Ansprüche, dadurch gekennzeichnet, daß die Projektionsvorrichtung eine Anzahl schmalbandiger Interferenzfilter (17) zur Erzeugung monochromatischer Strahlung enthält, die einzeln in den Strahlengang schaltbar sind.

9. Anordnung nach einem der vorhergehenden Ansprüche, dadurch gekennzeichnet, daß ein das Anregungsstrahlenbündel periodisch unterbrechender Chopper (30) vorgesehen ist, und daß die Signalauswertung mit der Unterbrecherfrequenz des Choppers (30) so gesteuert wird, daß das Fluoreszenzspektrum nur während der Zeit aufgenommen wird, in der das Anregungsstrahlenbündel unterbrochen ist.

## Revendications

1. Dispositif pour la mesure in vivo de l'opacification du cristallin, en particulier de la cataracte nucléaire, comprenant un dispositif pour le positionnement fixe de l'oeil à examiner, un dispositif de projection pour projeter l'image d'une fente sur le cristallin, un dispositif de mesure pour mesurer la lumière fluorescente stimulée sur le cristallin, un dispositif d'exploitation pour analyser la lumière fluorescente et un dispositif d'affichage pour les valeurs mesurées, caractérisé en ce que

a) le dispositif de projection (16—19) contient des moyens pour générer un faisceau de stimulation monochromatique dont la longueur d'onde $\lambda_A$ est comprise entre 350nm et 500nm,

b) le dispositif de mesure contient un spectrophotomètre enregistreur (22—26) qui enregistre, pour chaque longueur d'onde de stimulation $\lambda_A$, un spectre de fluorescence dans une région à plus grande longueur d'onde, comprise entre un minimum de 380nm et un maximum de 650nm,

c) le dispositif d'exploitation (29) détermine la longueur d'onde $\lambda_{max}$ coordonnée à l'intensité maximale I du spectre de fluorescence enregistré,

d) le dispositif d'exploitation (29) contient une mémoire avec une échelle de valeurs pour l'opacification du cristallin, à laquelle est coordonnée une table de valeurs, déterminée empiriquement, des paramètres de mesure $\lambda_A$ et $\lambda_{max}$, et

e) le dispositif d'exploitation (29) détermine la valeur recherchée de l'opacification du cristallin par comparaison des paramètres de mesure actuels $\lambda_A$, $\lambda_{max}$ avec la table de valeurs de la mémoire.

2. Dispositif selon la revendication 1, caractérisé en ce que

a) il comporte une commande à programme (28) qui ajuste successivement deux longueurs d'onde de stimulation différentes $\lambda_{A1}$ $\lambda_{A2}$ et déclenche l'analyse des spectres de fluorescence correspondants par le dispositif d'exploitation (29),

b) le dispositif d'exploitation (29) détermine les longueurs d'onde $\lambda_{max1}$ et $\lambda_{max2}$ coordonnées aux intensités maximales $I_1$, $I_2$ et forme en outre, à partir des intensités maximales $I_1$, $I_2$, le rapport d'intensité $I_1/I_2$ et

c) la table de valeurs, contenue dans la mémoire, comporte en outre des rapports d'intensité, déterminés empiriquement, pour des longueurs d'onde de stimulation correspondantes.

3. Dispositif selon la revendication 2, caractérisé en qu'une troisième longueur d'onde de stimulation $\lambda_{A3}$ est prévue, dont le spectre de fluorescence présente une intensité maximale $I_3$, que la commande à programme ajuste successivement les trois longueurs d'onde de stimulation $\lambda_{A1}$, $\lambda_{A2}$, $\lambda_{A3}$, que le dispositif d'exploitation forme, à partir des intensités maximales $I_1$, $I_2$, $I_3$ correspondantes, des rapports d'intensité $I_1/I_2$ et $I_2/I_3$ et que la table de valeurs de la mémoire est complétée en conséquence.

4. Dispositif selon une des revendications précédentes, caractérisé en ce que la longueur d'onde de stimulation $\lambda_A$ est formée par au moins une des longueurs d'onde 350nm, 395nm, 420nm, 470nm ou 500nm et les spectres de fluorescence correspondants sont situés dans les régions de longueurs d'onde 380—650nm, 430—650nm, 460—650nm, 490—650nm et 520—650nm.

5. Dispositif selon une des revendications précédentes, caractérisé en ce qu'un circuit pour numériser les spectres de fluorescence mesurés est coordonné au spectrophotomètre.

6. Dispositif selon une des revendications précédentes, caractérisé en ce qu'un circuit de réglage ajuste l'intensité du faisceau de stimulation en fonction de l'intensité de la lumière fluorescente.

7. Dispositif selon l'une quelconque des revendications précédentes, caractérisé en ce que l'axe optique (20) du faisceau de projection peut être incliné par rapport à l'axe (21) de l'oeil et de l'axe du faisceau de détection (27), de manière que des fluorescences parasites, excitées sur des couches tissulaires situées devant et derrière le cristallin (13), soient réduites autant que possible.

8. Dispositif selon une des revendications précédentes, caractérisé en ce que le dispositif de projection comporte un certain nombre de filtres d'interférence (17) à bande étroite pour la génération d'un rayonnement monochromatique, les filtres pouvant être interposés individuellement dans le faisceau.

9. Dispositif selon une des revendications précédentes caractérisé en ce qu'il comporte un pulsateur (30) qui interrompt périodiquement le faisceau de stimulation et que l'exploitation du signal est commandée avec la fréquence d'interruption du pulsateur (30), de manière que le spectre de fluorescence soit seulement enregistré pendant le temps où le faisceau de stimulation est interrompu.

**Claims**

1. An apparatus for making an in vivo measurement of eye lens cloudiness such as caused by cataracta nuclearis, the eye lens having an inherent natural fluorescence corresponding to the cloudiness, the apparatus comprising:

a device for positioning the head of the patient so as to place the eye in a predetermined fixed position desirable for making the in vivo measurement;

a projecting device for projecting a slit image onto the eye lens, the projecting device including:

a light source for generating a monochromatic excitation beam of light defining an excitation beam axis and having a wavelength $\lambda_A$ lying in the range of 350nm to 500nm;

imaging means arranged in said beam for forming the slit image; and

optic means for focusing the slit image on the eye so as to excite the natural fluorescence in the eye lens to produce a fluorescence light defining a fluorescence light axis;

a measuring device for measuring said fluorescence light, the measuring device including:

a recording spectrophotometer for recording a fluorescence spectrum in the wavelength range of 380nm to 650nm which is longer than said excitation wavelength $\lambda_A$;

optic directing means for receiving said fluorescence light from the eye lens and directing the same to said recording spectrophotometer; and,

signal processing means for analyzing the fluorescence spectrum recorded by said recording sprectrophotometer to determine the wavelength $\lambda_{max}$ corresponding to the maximum intensity I of said recorded fluorescence spectrum; said signal processing means including:

a memory having a scale of values for eye lens cloudiness and storing an empirically determined table of values of the measured parameters ($\lambda_A$ and $\lambda_{max}$) corresponding to said scale of values; and, comparator means for comparing the actual measured parameters $\lambda_A$, $\lambda_{max}$ to said table of values thereby determining the degree of the eye lens cloudiness.

2. The apparatus of claim 1, comprising; program control means for successively setting two

different excitation wavelengths ($\lambda_{A1}$ and $\lambda_{A2}$) and initiating the analysis of the spectra corresponding to said excitation wavelengths by said signal processing means which determines the wavelengths ($\lambda_{max1}$ and $\lambda_{max2}$) corresponding to the maximum intensities ($I_1$ and $I_2$) and forms an intensity ratio ($I_1/I_2$) from said maximum intensities ($I_1$ and $I_2$); and, said table of values stored in said memory also including empirically determined intensity ratios for corresponding excitation wavelengths.

3. The apparatus of claim 2, wherein: a third excitation wavelength $\lambda_{A3}$ is provided having a fluorescence spectrum with a maximum intensity $I_3$, said program control means successively sets the three excitation wavelengths ($\lambda_{A1}$, $\lambda_{A2}$, $\lambda_{A3}$) and said signal processing means forms intensity ratios $I_1/I_2$ and $I_2/I_3$ from said maximum intensities ($I_1$, $I_2$, $I_3$) corresponding thereto; and said table of values is supplemented with said intensity ratios.

4. The apparatus of claim 3, wherein: at least one of the wavelengths 350nm, 395nm, 420nm, 470nm or 500nm acts as the excitation wavelength $\lambda_A$; and, the fluorescence spectra corresponding to said wavelengths $\lambda_A$ lie in the wavelength regions 380—650nm, 430—650nm, 460—650nm, 490—650nm and 520—650nm, respectively.

5. The apparatus of claim 4, said measuring device including a circuit arrangement for digitalizing the fluorescence spectra measured by the spectrophotometer.

6. The apparatus of claim 1, comprising: control circuit means for adjusting the intensity of the excitation beam of light in dependence on the intensity of the fluorescence light.

7. The apparatus of claim 1, wherein the eye lens defines an eye lens axis, and, said excitation beam axis being inclined with respect to said eye lens axis and said fluorescence light axis so as to cause interfering fluorescence excited in tissue layers lying in front of and in back of the eye lens to be reduced as far as possible.

8. The apparatus of claim 1, said light source including a plurality of narrow band interference filters individually placeable into the path of said excitation beam for producing the monochromatic illumination.

9. The apparatus of claim 1, said projecting device including:
chopper for periodically interrupting said excitation beam and wherein the signal evaluation is controlled with the interrupter frequency of the a chopper so that the fluorescence spectrum is recorded only during the time in which the excitation beam of light is interrupted.

Fig.1

Fig.2

Fig.3

# Fig.4